# EUROPEAN PATENT SPECIFICATION

(11) **EP 4 162 015 B1**
(45) Date of publication and mention of the grant of the patent: **14.05.2025**
(21) Application number: 21734551.1
(22) Date of filing: 08.06.2021
(51) Int. Cl.: C10M 141/02, A61K 8/06, B27K 3/15

(54) **MICROEMULSIONS AND THEIR USES**
MIKROEMULSIONEN UND IHRE VERWENDUNGEN
MICROÉMULSIONS ET LEURS UTILISATIONS

(30) Priority: 09.06.2020 SE 2050672
(43) Date of publication of application: 12.04.2023
(73) Proprietor: Biobase Sweden AB, 120 40 Årsta (Stockholm) (SE)
(72) Inventor: WIKLUND, Per, 117 60 STOCKHOLM (SE)
(74) Representative: Novitas Patent AB
(86) International application number: PCT/SE2021/050548
(87) International publication number: WO 2021/251883

(56) References cited:
- EP-A1- 1 627 032
- EP-A1- 3 308 766
- US-A1- 2008 031 906

## Description

### Field of invention

The invention relates to microemulsions, and in particular to non-water-soluble microemulsions.

### Background

Oils are efficient lubricants to reduce friction between moving parts in many mechanical devices, but oils are by nature liquids that must be contained to stay put. In contrast lubricant greases are viscoelastic materials that do not flow away unless high pressure is applied. Hence lubricant greases can be used in e.g. bearings, where they can be contained efficiently with gaskets. Lubricant greases, and high viscosity oils, are also commonly applied to moving mechanical parts that cannot be enclosed, such as driving chains and belts, wire winches, cranes, braking calipers, leaf springs, chain saw chains etc. In such applications it is inevitable that lubricant is emitted to the environment, and that the lubricant gets into contact with water or moisture. For these reasons it is preferable if the lubricant is not environmentally harmful and is not broken down or washed away by water.

Lubricant greases consist mainly of oil with a minor content of a thickener. The thickener can be fine powders, yielding greases that are suspensions. Most commonly however, oil is thickened by emulsification with metal soaps of fatty acids. The most common fatty acids used are stearic acid and 12-hydroxy-stearic acid, usually saponified with lithium or calcium as counterions. Lithium has of late found another use in electric batteries, and competition for the commodity has increased. The oils used in lubricant greases are usually mineral oils of considerable viscosity, but it is also possible to use vegetable oils at the price of poor oxidation stability and poor low temperature properties.

Microemulsions are seemingly homogenous mixtures of liquids not normally miscible with each other due to their vast difference in polarity. In the presence of surfactants such liquids can in certain cases, and in certain proportions, spontaneously form visibly clear emulsions, in particular in presence of so-called co-surfactants which are usually medium sized alcohols. Like macroemulsions, which are opaque due to larger micelle size, microemulsions can be of oil-in-water or water-in-oil types. In addition, microemulsions can appear in a third discontinuous type.

Descriptions of emulsions and microemulsions for use as lubricants are abundant in the patent literature, in particular in the field of metal working fluids where the general aim is to incorporate as much water as possible in the fluid (for the purpose of cooling) yet maintain the lubricant effect. One example is EP 1627 032, which discloses emulsified grease for lubrication, said grease comprising glycerol.

In general, the described types of emulsions consist of mineral oils, often of high viscosity, water (often more than 25 w%), and emulsifier(s). The content of emulsifier(s) is usually kept as low as possible as the cost for many efficient synthetic emulsifiers is relatively high. Hence, there is a need for new microemulsions reducing the need for costly emulsifiers, and reducing the environmental impact through the use of oils or hydrocarbons of non-fossil origin.

### Short Description of the invention

The invention relates to a microemulsion comprising
water in an amount of 1-30 w%;
sodium or potassium oleate, Na/K salts of tall oil fatty acid, and/or Na/K salts of C16-C18 saturated or unsaturated fatty acids in an amount of 10-40 w%;
oleic acid, tall oil fatty acid, or C16-C18 saturated or unsaturated fatty acids in an amount of 2-40 w%;
ethanol in an amount of 5-40 w%;
glycerol in an amount of 5-40 w%;
and liquid hydrocarbon(s) in an amount of 5-40 w%, up to a maximum or total of components parts of 100 w%.

The microemulsion is prepared using a method comprising the following consecutive steps:
(a) mixing of NaOH or KOH with water, glycerol, and ethanol to obtain a solution;
(b) mixing oleic acid, tall oil fatty acid, and/or C16-C18 saturated or unsaturated fatty acid(s) into the solution to obtain a microemulsion;
(c) mixing of the microemulsion with liquid hydrocarbon(s) to form a second microemulsion;
(d) optionally addition of water or ethanol;
(e) optionally partial evaporation of the ethanol,
whereby steps (d) and (e) may be performed in reversed order.

The microemulsion finds its use as e.g. a lubricant, metal working fluid, water proofing agent for porous materials, and in in pharmaceutical preparations.

The microemulsions according to the invention comprise low viscosity hydrocarbon fluids (biobased or of fossil origin), high amounts of natural emulsifiers, low amounts of water, and glycerol. The innovative microemulsions as described herein possess a unique specific property in that they form a non-soluble material when contacted with excess water. That is to say, they cannot be referred to as *soluble oils* which is a common term for oil preparations forming emulsions when mixed with water.

The microemulsions disclosed herein exist as microemulsions at ambient temperature, unless another temperature is specifically disclosed. In certain embodiments, microemulsions exist as microemulsions only at a temperature exceeding 60°C, e.g. in a temperature interval from 60 to 90°C. Microemulsions comprising palmitic and/or stearic acid are examples of the latter kind of microemulsions.

The invention shall now be further described with reference to the appended claims set, wherein independent claims constitute aspects of the invention and dependent claims denote preferred embodiments.

### Detailed Description of the Invention

It has unexpectedly been found that certain homogenous clear mixtures of water, water soluble alcohols, sodium oleate, oleic acid and glycerol, form homogenous mixtures when blended with non-polar liquids such as hydrocarbon solvents and oils.

Furthermore, the amount of such non-polar liquids that can be added to the original mixture, still yielding a clear homogenous mixture, can be increased by the addition of more oleic acid. These homogenous mixtures are microemulsions, i.e. thermodynamically stable emulsions that spontaneously form without the need for high shear mixing (the process of homogenizing otherwise immiscible liquid phases) associated with the formation of ordinary opaque (macro) emulsions.

Depending on the composition and order of addition of the liquid components, these microemulsions behave differently when diluted with more water. In certain cases, were the amount of water present when the microemulsion is formed is small, dilution with water yields a fluffy or crystalline-like material which does not dissolve in water. In other cases, in particular when the amount of water present during the formation of the microemulsion is higher, dilution with water yields soapy emulsions easily washed away with water.

It was observed that when a solution of oleic acid (54 g) and hexadecane (33 g) was added by slight stirring to a mixture of aqueous sodium hydroxide (18 g 33% NaOH), glycerol (69 g) and ethanol (60 g 95%), an opaque mixture was formed. Upon addition of a portion of oleic acid (5 g) the mixture became a clear microemulsion. Only 42 g of oleic acid was needed to neutralize the sodium hydroxide, meaning that about 17 g of oleic acid, which is oil soluble, resides in the mixture. Another addition of hexadecane (10 g) again made the mixture opaque, and a further addition of oleic acid (5 g) again produced a microemulsion. This scheme of gradual addition of the two components could be continued with more hexadecane (up to at least another 35 g) and more oleic acid (up to at least 10 g) to maintain the microemulsion state of the system. When a portion of the final microemulsion (2.5 g) was poured on water (100 g), a fluffy white crystalline-like material was formed which had the consistency of a soft lubricant grease. When smeared on fingers this material was not possible to wash away with water without the use of a detergent.

To further analyze the behavior of this system, five different experimental mixtures were prepared, see Table 1.

**Table 1.**

| **Case** | **1** | **2** | **3** | **4** | **5** |
|---|---|---|---|---|---|
| NaOH (g) | 6 | | 6 | 6 | 6 |
| Water (g) | 12 | 12 | 12 | 12 | 60 |
| Glycerol(g) | 70 | 70 | 130 | | 70 |
| Ethanol (g) | 60 | 60 | | 130 | 60 |
| Oleic acid (g) | 70 | 70 | 70 | 70 | 70 |
| Description of mixture | Homogenous clear liquid | Opaque separating mixture | Thick slightly opaque mixture | Homogenous clear liquid | Homogenous clear liquid |

In Case **1,** a solution of sodium hydroxide, a small quantity of water (deionized), glycerol and ethanol (95%), formed a homogenous clear microemulsion upon light stirring wherein the remaining oleic acid not neutralized by sodium hydroxide (to form sodium oleate) constitutes the oil phase. Whereas sodium oleate has a limited solubility in water, oleic acid is practically insoluble in water. And although the protonated carboxy group of oleic acid is in itself polar, it is not polar enough for (non-ionized) oleic acid to behave as a surfactant. Hence, as shown in Case **2,** in the absence of the strong base sodium hydroxide, no emulsion was formed.

If the portion of ethanol is fully replaced by glycerol, as in Case **3,** the resulting mixture appears homogenous, albeit not fully clear. If glycerol is fully replaced by ethanol, as in Case **4,** the mixture forms a clear microemulsion. It was also found that a microemulsion was formed when more water was used to dissolve the sodium hydroxide, as shown in Case **5.** The microemulsions of Cases **1, 3, 4** and **5** all behave similar to washing soap when mixed with water, i.e. they lather and can easily be rinsed away with water.

In Case **1** it was possible to add a portion of hexadecane (70 g) to form a new second generation clear stable microemulsion. Hexadecane (70 g) could also be added in Case 3, but the resulting emulsion was only formed after vigorous shaking and was not entirely clear. Upon storage for several weeks this very viscous emulsion started to separate, leading to the conclusion that it was not a true microemulsion (which would be stable). In Case 5, a portion of up to 25 g of hexadecane could be added leading to formation of a second generation microemulsion.

Hence the presence of glycerol is necessary for the second generation microemulsions to form. The presence of ethanol is not necessary for formation of second generation emulsions in general (after addition of hexadecane) but appears to be necessary for the formation of a (stable and visibly clear) microemulsion and also contributes to ease of blending of the components as its presence markedly reduces viscosity.

The ability of oleic acid/sodium oleate in water or glycerol, to form emulsions with hexadecane, is known [1], but apparently only macroemulsions were studied, since no mention of microemulsions was made and high energy mixing by sonication was used to prepare the *white* emulsions. The authors did not report any use of ethanol or other cosolvents, nor the presence of both glycerol and water simultaneously.

Microemulsions consisting of sodium oleate/oleic acid, water and isopropanol have also been reported [**2**], but further addition of hydrocarbon solvents or oils to the mixtures has not been disclosed.

It was found that two of these three second generation microemulsions (stemming from Cases **1** and **3**) gave the grease-like material when poured on water (cold or hot). The sticky material could not be washed away or dissolved in much larger quantities of water.

This behavior is surprising for several reasons. Sodium soaps of fatty acids are generally not useful as thickeners in lubricating greases because they are water soluble (compare ordinary washing soap) and give greases that are easily dissolved and washed away by water [**3**]. Furthermore, production of water-resistant lubricating greases by conventional methods requires oils of much higher viscosity and hence molecular size than the C16-structure of hexadecane. Lastly, oleic acid, although extremely abundant in natural fats and oils, is not normally thought of as a basic component of lubricating greases.

A microemulsions displaying the behavior of formation of grease-like floccular material when poured on water will in the following be referred to as a *Flocculation Forming Microemulsion* (FFMe).

When the emulsion stemming from Case **5** was poured on water it instead gave a soapy material easily washed away with water. This microemulsion, initially containing much more water, is likely an oil-in-water type microemulsion, whereas the FFMe stemming from Case **1** is likely to be a water-in-oil microemulsion. A further observation was that the latter FFMe could be diluted with at least 60 g of (deionized) water and still retain the FFMe characteristics.

The invention shall now be described with reference to the below examples, which shall not be seen as any limitation on the claimed scope whatsoever. The person skilled in the art realizes that exchange of component parts and change of constitution may be made, without departing from the inventive concept.

### Delineating characteristics

To further delineate important characteristics of the above described microemulsions leading to the flocculation forming properties, a series of experiments were performed. In the following the definition of an FFMe is that when 3 ml of the microemulsion is poured on 100 ml water, slight swirling of the vessel containing the mixture generates a floating (white) floccular material within 2 hours. In cases of strong FFMe-effect the material formed is granular or clearly lumpy.

| **Case#** | **6** | **7** | **8** | **9** | **10** | **11** | **12** | **13** | **14** |
|---|---|---|---|---|---|---|---|---|---|
| NaOH (g) | 6 | 6 | 6 | 6 | 6 | 6 | 6 | 6 | 6 |
| Water (g) | 12 | 12 | 12 | 12 | 12 | 12 | 12 | 12 | 12 |
| Glycerol (g) | 20 | 20 | 20 | 40 | 60 | 60 | 20 | 20 | 60 |
| EtOH (g) | 20 | 20 | 20 | 20 | 60 | 60 | 60 | 30 | 60 |
| Oleic Acid (g) | 50 | 60 | 60 | 60 | 60 | 70 | 70 | 70 | 70 |
| Hexadecane (g) | 10 | 10 | 30 | 30 | 30 | 30 | 30 | 30 | 10 |
| Clear | No | Almost | Yes | Yes | Yes | Yes | Yes | No | Yes |
| FFMe-effect | No | No | No | Slight | No | Strong | Moderate | No | No |

In all cases above (**6-14**) the initial mixing of NaOH, water, glycerol and ethanol gives a clear solution. When exchanging ethanol for isopropanol this was not found to be the case even if the final microemulsions after addition of hexadecane did form. However, the isopropanol containing microemulsions did not show FFMe-behavior in any case.

Microemulsions could also be prepared with Rape seed methyl ester (RME/FAME) and vegetable oils, but these did not show the FFMe-effect.

### Examples

Using the methodology described above, a series of FFMe:s were prepared. Table 3 shows examples of FFMe:s based on hexadecane. The viscosity of the fluid in **Example 1** was found to be 30 cp at 20 °C, and the viscosity of the fluid in **Example 2** was found to be 65 cp at 20 °C.

**Table 3 Hexadecane-based FFMe:s**

| **Example** | **1** | **2** | **3** | **4** | **5** | **6** | **7** |
|---|---|---|---|---|---|---|---|
| Water w% | 5 | 5 | 9 | 6 | 3 | 8 | 7 |
| Ethanol w% | 21 | 19 | 12 | 25 | 26 | 25 | 30 |
| Glycerol w% | 24 | 31 | 24 | 25 | 26 | 25 | 10 |
| Sodium Oleate w% | 16 | 14 | 27 | 19 | 20 | 19 | 23 |
| Oleic Acid w% | 10 | 10 | 10 | 12 | 12 | 11 | 14 |
| Hexadecane w% | 24 | 22 | 18 | 13 | 13 | 12 | 15 |

Table 4 shows five examples where HVO (hydrotreated vegetable oil) is used as a component of FFMe:s. HVO is most commonly used as a high quality renewable diesel, but can also be used as a chemical component of various functional fluids. Because of its chemical origin in fatty acids, HVO is dominated by C16-C18 hydrocarbons, but unlike hexadecane it is highly isomerized. **Example 10** shows that potassium oleate works as well as sodium oleate.

As stated above rape seed methyl ester (RME/FAME) and vegetable oils can give stable microemulsions, but these did not show the FFMe-effect. However, an FFMe containing a combination of HVO and rapeseed oil could be formulated **(Example 11),** and similarly a formulation with linseed oil was made to which a portion of wood tar could be added with retained FFMe-behavior **(Example 12).** The latter example shows that the FFMe-formulations can dissolve other components and incorporate them into the microemulsion.

The microemulsion of Example 6 could be further diluted with up to 35 w% water or 30 w% ethanol without compromising the microemulsion state of the preparation. At higher dilution, translucency ceased and eventually the mixture formed several liquid phases.

**Table 4 HVO-based FFMe:s**

| **Example 8** | **Example 9** | **Example 10** | **Example 11** | **Example 12** |
|---|---|---|---|---|
| 6 w% water | 6 w% water | 6 w% water | 7 w% water | 7 w% water |
| 23 w% ethanol | 23 w% ethanol | 23 w% ethanol | 20 w% ethanol | 18 w% ethanol |
| 26 w% glycerol | 26 w% glycerol | 26 w% glycerol | 19 w% glycerol | 18 w% glycerol |
| 20 w% Sodium Oleate | 20 w% Sodium UFA salt | 20 w% Potassium Oleate | 22 w% Sodium UFA salt | 21 w% Sodium UFA salt |
| 12 w% oleic acid | 12 w% UFA | 12 w% oleic acid | 13 w% UFA | 13 w% UFA |
| 13 w% HVO | 13 w% HVO | 13 w% HVO | 12 w% HVO | 11 w% HVO |
| | | | 7 w% rapeseed oil | 7 w% linseed oil |
| | | | | 5 w% wood tar |

UFA is short for C16-C18 unsaturated fatty acids (CAS# 67701-08-8). HVO is short for hydrotreated vegetable oil (diesel type fraction), REACH-registration number 01-2119450077-42-0000

Further examples of FFMe:s based on other hydrocarbon liquids are found in Table 5. Noteworthy is that microemulsification of the PAO-based engine oil 5W-30 requires more free oleic acid. This is a parallel to the initial observation that higher amounts of hexadecane could be microemulsified with increased addition of oleic acid.

**Table 5 Further FFMe:s**

| **Example 13** | **Example 14** | **Example 15** | **Example 16** | **Example 17** |
|---|---|---|---|---|
| 6 w% water | 7 w% water | 5 w% water | 7 w% water | 5 w% water |
| 23 w% ethanol | 21 w% ethanol | 22 w% ethanol | 10 w% ethanol | 7 w% ethanol |
| 26 w% glycerol | 20 w% glycerol | 22 w% glycerol | 28 w% glycerol | 31 w% glycerol |
| 20 w% Sodium UFA salt | 23 w% Sodium UFA salt | 16 w% Sodium Oleate | 23 w% Sodium UFA salt | 16 w% Sodium UFA salt |
| 12 w% UFA | 14 w% UFA | 24 w% oleic acid | 14 w% UFA | 16 w% UFA |
| 13 w% ISA | 15 w% T9 | 11 w% 5W30 | 18 w% HVO | 25 w% HVO |

UFA is short for C16-C18 unsaturated fatty acids (CAS# 67701-08-8).

ISA is short for C11-C15 isoalkanes (CAS# 90622-58-5).

T9 is a napthenic base oil of viscosity 9 mm²/s at 40 °C (Nynas AB).

5W-30 is a formulated PAO-based engine oil.

### Use of additional fatty acids

In a variant of the preparation of **Example 16,** 18 g of aqueous sodium hydroxide (33%) was diluted with 50 g of ethanol and 55 g of glycerol, after which 70 g of Tall Oil Fatty Acids (TOFA) was mixed in, followed by addition of 35 g of HVO. This procedure gave a dark colored microemulsion. The contents of TOFA are dominated by C18 unsaturated fatty acids.

In yet another variant of **Example 16,** 18 g of aqueous sodium hydroxide (33%) was diluted with 30 g of ethanol and 60 g of glycerol after which a solution of 10 g of palmitic acid in 60 g of oleic acid was mixed in together with 35 g of hexadecane to form a microemulsion.

In yet another variant of **Example 16,** 18 g of aqueous sodium hydroxide (33%) was diluted with 30 g of ethanol and 60 g of glycerol, after which 70 g molten palmitic or stearic acid mixed with 35 g of hexadecane was added under vigorous stirring. While the resulting preparation was warm, it showed the same microemulsion characteristics as all of the above examples, but when cooled to room temperature the preparation solidified into a uniform wax like material with a melting point of about 60 °C.

### Behavior of the flocculation forming microemulsions (FFMEs)

The floccular/granular grease-like material formed when the microemulsions were poured on proportionally high amounts of water was possible to capture in a sieve of mesh size 1 mm at room temperature upon which the sieve was clogged and no further flow through it occurred.

When the microemulsions were mixed with about equal volumes of water, thick creamy emulsions formed. These emulsions were found to be stable over a period of several days, but as water (and ethanol) evaporated the material gradually changed into a clear oil-like substance of high viscosity.

When the ethanol in microemulsion of **Example 8** was allowed to fully evaporate, what resulted was a very viscous oil-like substance with a density of 0.97 g/ml at 20 °C, and a dynamic viscosity at 40 °C of around 220 cp, indicating a kinematic viscosity of about 227 mm²/s at 40 °C. The substance was immiscible with water, but gradually formed a similar grease-like material as in the cases when an FFMe was poured on water (see above), but of a lumpier texture. Furthermore, when the microemulsion of Example 16 was heated to 80 °C until bubbling had subsided and the ethanol had been evaporated, a microemulsion of very high viscosity remained (987 cp at 20 °C).The high viscosity microemulsion retained its stability over a period of 3 months. In a subsequent step, the new microemulsion was heated to 100 °C until bubbling had subsided and the water had been evaporated, whereby a microemulsion of viscosity 565 cp at 20 °C remained.

The microemulsion of Example 16 may be used as a thickener of HVO. When 50 w% of the said microemulsion was shaken with HVO, a gel-like substance was produced with a viscosity of 52 cp at 20 °C. Similarly, 35% w% of the microemulsion yielded a gel-like substance of viscosity 22 cp at 20 °C. The gelated HVO may be used as a fuel or as a lubricant.

### Applications of the microemulsions

Because of the grease-like behavior of these microemulsions after contact with water, they can be used for lubrication of open mechanical systems exposed to the weather such as the sliding parts of hydraulic booms and cranes, wires and winches, driving belts and chains, chain saw chains, sliding tables or ramps etc. This type of equipment is very common on ships' decks. In the wet the material would act similarly to common lubricant grease, and when allowed to dry out the material instead resembles a high viscosity oil.

Another application for the microemulsions are as metal working fluids. In all these lubricant cases it is suitable to additivate the liquid to preserve the function over time with phenolic (such as BHT) and/or aromatic amine antioxidants, and to improve lubricity with antiwear additives (such as zink dithiophosphates and glycerol monooleate) and extreme pressure additives (such as dithiocarbamates).

The microemulsions can be used to protect metal surfaces from corrosion. A plate mild steel was dipped into the microemulsion of **Example 8,** after which the plate was placed in a beaker containing an aqueous solution of sodium chloride (4 %). As comparison a plate from the same sheet of steel was also placed in the same solution. After 48 hours the plates were lifted from the brine solution. The treated plate was still covered by the grease-like layer. The plates were washed with detergent and clean water. Upon inspection after the cleaning the untreated plate was found to have very clear corrosion scars whereas the treated plate was in pristine condition. Easily removable layered corrosion protection is often referred to as temporary corrosion protection and is useful e.g. during storage (hours to months) of partially processed metal parts to be ready for the next stage of production.

It was found that when emulsions of the invention are painted onto raw concrete surfaces, such as e.g. concrete slabs, what results is a persistent water repellant surface. If a drop of water is applied to a raw concrete surface it is normally instantly absorbed into the material. In contrast, a drop of water applied to a raw concrete surface treated with a microemulsion of the invention stays intact and rolls off upon tilting. Larger amount of water sprayed onto the treated concrete surface triggers the formation of the grease-like material making the surface sticky. As the experiment with steel plates shows, the sticky grease-like layer is impenetrable to water. In combination with the observation that the floccular material formed when the FFMe:s are poured on water, it is envisioned that they can function as water proofing agent on porous materials like rock or concrete.

It was also found that when paper, paperboard or cardboard was coated with a thin layer, e.g. 1-100 µm, of the innovative microemulsion, the coating provided a measure of waterproofing. Hence, the microemulsions are useful e.g. as barrier for packaging materials.

In a similar fashion the FFMe:s can be used for impregnation of wood, both to make it water-repellant and more resistant to rot. An FFMe with a dissolved drying (or non-drying) vegetable oil and wood tar, possibly in combination with conventional wood preservatives including sulfur, silicates, copper salts/complexes or (suspended) metallic copper particles and methylisothiazolinones may be produced. In an exemplary embodiment, two dimensionally identical pieces (75x17x195 mm) of Nordic spruce was cut from the same plank at the same time, ensuring equal moisture content. One of the pieces was put horizontally with the largest area down in a container with a layer of the microemulsion of **Example 12** reaching about 5 mm up the side of the wooden piece. After 24 h the piece was turned to soak the other side. After another 24 h the piece was lifted from the bath and allowed to dry. The two pieces of wood were stored indoors for one week after which weighing showed an increase in weight for the treated piece by 4% compared to the untreated piece. When sawn apart, the cross-section of the treated piece showed the liquid had penetrated about 2-5 mm into the wood, and the surface of the wood had fatty feel to it even after rinsing with water and letting dry. In a study from 2006 [4] it was concluded that (macro) emulsions of tall oils can be used efficiently to impregnate wood. The limiting factor was found to be that the relatively large micelles of the emulsions was filtered by the cellulose of the wood, and hence deep penetration was difficult. A microemulsion of the invention would suffer much less from this problem, in particular if the viscosity is kept low with the content of ethanol.

It was found that the microemulsions may be used as an alternative to silicone-based products to rejuvenate and add shine to rubber surfaces such as car tires. Similarly, the microemulsions find use as a leather shine and impregnation fluid.

### Literature references

[**1**] "Self-assembly and emulsions of oleic acid-oleate mixtures in glycerol", Delampe, Jerome, Barrault, Douliez; Green Chemistry, 2011 (13), 64-68, https:!/doi.org/10.1039/C0GC00452A]
[**2**] "Conversion of a surfactant-based microemulsion to a surfactant-free microemulsion"; Liu, Lu, Zhang, Zhu, Huang; Soft Matter, 2019 (15), 462-469, https:!/doi.org/10.1039/C8SM02444H]
[**3**] Chemistry and Technology of Lubricants, 3:rd edition: ed. Mortier, Fox, Orszulik: Springer, Dordrecht Heidelberg, London, New York: 2010: p. 420, https://link.springer.com/book/10.1007/978-1-4615-3554-6.
[**4**] "Tall oil/water - emulsions as water repellents for Scots pine sapwood" Hyvönen, Piltonen, Niinimäki, Holz als Roh- und Werkstoff, 2006 (64), 68-73 https://doi.org/10.1007/s00107-005-0040-5.

The invention relates to a microemulsion comprising
water in an amount of 1-30 w%;
sodium or potassium oleate, Na/K salts of tall oil fatty acid, and/or Na/K salts of C16-C18 saturated or unsaturated fatty acids in an amount of 10-40 w%;
oleic acid, tall oil fatty acid, or C16-C18 saturated or unsaturated fatty acids in an amount of 2-40 w%;
ethanol in an amount of 5-40 w%;
glycerol in an amount of 5-40 w%;
and liquid hydrocarbon(s) in an amount of 5-40 w%, up to a maximum or total of components parts of 100 w%.

Moreover, methods of manufacture and uses of the microemulsion are disclosed.

## Claims

1. Microemulsion comprising
water in an amount of 1-30 w%;
sodium or potassium oleate, Na/K salts of tall oil fatty acid, and/or Na/K salts of C₁₆-C₁₈ saturated or unsaturated fatty acids in an amount of 10-40 w%;
oleic acid, tall oil fatty acid, or C₁₆-C₁₈ saturated or unsaturated fatty acids in an amount of 2-40 w%;
ethanol in an amount of 5-40 w%;
glycerol in an amount of 5-40 w%;
and liquid hydrocarbon(s) in an amount of 5-40 w%, up to a maximum or total of components parts of 100 w%.

2. Microemulsion according to claim 1, comprising
water in an amount of 2-10 w%;
sodium or potassium oleate , Na/K salts of tall oil fatty acid, and/or Na/K salts of C₁₆-C₁₈ saturated or unsaturated fatty acids in an amount of 14-27 w%;
oleic acid, tall oil fatty acid, or C₁₆-C₁₈ saturated or unsaturated fatty acids in an amount of 10-24 w%;
ethanol in an amount of 5-40 w%, e.g. 5-26 w%;
glycerol in an amount of 10-31 w%;
and liquid hydrocarbons in an amount of 11-24 w%, up to a maximum or total of components parts of 100 w%.

3. Microemulsion according to any of the preceding claims, wherein the liquid hydrocarbon is C₁₁-C₂₀ alkanes and/or isoalkanes.

4. Microemulsion according to any of the preceding claims, wherein the liquid hydrocarbon is hexadecane, diesel, HVO, PAO, a naphthenic or paraffinic base oil or a combination thereof.

5. Microemulsion according to any of the preceding claims, having a viscosity in the interval of from 20 to 400 cp at 20 °C.

6. Microemulsion according to any of the preceding claims, having a storage stability of more than 30 days at 20 °C.

7. Microemulsion according to any of the preceding claims, to which wood tar has been added in an amount of up to 15 w%.

8. Microemulsion according to any of the preceding claims, to which an antioxidant, antiwear agent, and/or extreme-pressure additive has been added in an amount of 0.1 to 5 w%.

9. Microemulsion according to any of the preceding claims, to which wood preservatives such as antifungal substance(s) have been added in an amount of 0.1 to 5 w%.

10. Microemulsion according to any of the preceding claims, comprising 12-26 w% ethanol.

11. Gel, comprising 35-50 w% of the microemulsion according to any of claims 1 - 10, and 50 - 65 w% of a hydrotreated vegetable oil, up to a maximum or total of components parts of 100 w%.

12. Method of preparing a microemulsion according to any of claims 1 - 10, comprising the following consecutive steps:
(a) mixing of NaOH or KOH with water, glycerol, and ethanol to obtain a solution;
(b) mixing oleic acid, tall oil fatty acid, and/or C₁₆-C₁₈ saturated or unsaturated fatty acid(s) into the solution to obtain a microemulsion;
(c) mixing of the microemulsion with liquid hydrocarbon(s) to form a second microemulsion;
(d) optionally addition of water or ethanol;
(e) optionally partial evaporation of the ethanol,
whereby steps (d) and (e) may be performed in reversed order.

13. Method according to claim 12, wherein in step (d) water or ethanol is added.

14. Method according to claim 12, wherein in step (e) ethanol is partially evaporated.

15. Use of a microemulsion according to any of claims 1 - 10 for temporary corrosion protection on metal surfaces, especially steel.

16. Use of a microemulsion according to any of claims 1 - 10 as a lubricant.

17. Use according to claim 16, wherein driving chains, chain saw chains, belts, wire winches, cranes, brake calipers, chain saw chains, or leaf springs are lubricated.

18. Use of a microemulsion according to claims 1-10 as metal working fluid.

19. Use of a microemulsion according to any of claims 1 - 10 as a water proofing agent for porous materials.

20. Use according to claim 19, wherein the porous materials constitute rock cavities and/or concrete constructions.

21. Use of a microemulsion according to any of claims 1 - 10 for coating of paper, paperboard, cardboard, or rubber.

22. Use of a microemulsion according to any of claims 1 - 10 as a leather or rubber shine liquid, or as a leather impregnation liquid.

23. Use of a microemulsion according to any of claims 1 - 10, as a thickening agent for hydrotreated vegetable oil.

24. Use of a gel according to claim 11 as a fuel or a lubricant.

25. Wood block, **characterized in that** it is impregnated with a microemulsion according to any of claims 1 - 10.

## Patentansprüche

1. Mikroemulsion, umfassend
Wasser in einer Menge von 1-30 Gewichts-%;
Natrium- oder Kaliumoleat, Na/K-Salze von Tallölfettsäuren und/oder Na/K-Salze von gesättigten oder ungesättigten C₁₆-C₁₈-Fettsäuren in einer Menge von 10-40 Gewichts-%;
Ölsäure, Tallölfettsäure oder gesättigte oder ungesättigte C₁₆-C₁₈-Fettsäuren in einer Menge von 2-40 Gewichts-%;
Ethanol in einer Menge von 5-40 Gewichts-%;
Glycerin in einer Menge von 5-40 Gewichts-%;
und flüssigen Kohlenwasserstoff(e) in einer Menge von 5-40 Gewichts-%, bis zu einem Maximum oder einer Summe von Bestandteilen von 100 Gewichts-%.

2. Mikroemulsion nach Anspruch 1, umfassend:
Wasser in einer Menge von 2-10 w%;
Natrium- oder Kaliumoleat, Na/K-Salze von Tallölfettsäuren und/oder Na/K-Salze von gesättigten oder ungesättigten C₁₆-C₁₈-Fettsäuren in einer Menge von 14-27 Gewichts-%;
Ölsäure, Tallölfettsäure oder gesättigte oder ungesättigte C₁₆-C₁₈-Fettsäuren in einer Menge von 10-24 Gewichts-%;
Ethanol in einer Menge von 5-40 Gewichts-%, z. B. 5-26 Gewichts-%;
Glycerin in einer Menge von 10-31 Gewichts-%;
und flüssige Kohlenwasserstoffen in einer Menge von 11-24 Gewichts-%, bis zu einem Maximum oder einer Summe von Bestandteilen von 100 Gewichts-%.

3. Mikroemulsion nach einem der vorherigen Ansprüche, wobei der flüssige Kohlenwasserstoff C₁₁-C₂₀-Alkane und/oder Isoalkane ist.

4. Mikroemulsion nach einem der vorherigen Ansprüche, wobei der flüssige Kohlenwasserstoff Hexadecan, Diesel, HVO, PAO, ein naphthenisches oder paraffinisches Grundöl oder eine Kombination davon ist.

5. Mikroemulsion nach einem der vorherigen Ansprüche, die eine Viskosität in dem Bereich von 20 bis 400 cP bei 20 °C aufweist.

6. Mikroemulsion nach einem der vorherigen Ansprüche, die eine Lagerstabilität von mehr als 30 Tagen bei 20 °C aufweist.

7. Mikroemulsion nach einem der vorherigen Ansprüche, zu der Holzteer in einer Menge von bis zu 15 Gewichts-% hinzugefügt worden ist.

8. Mikroemulsion nach einem der vorherigen Ansprüche, zu der ein Antioxidans, ein Antiverschleißmittel und/oder ein Hochdruckadditiv in einer Menge von 0,1 bis 5 Gewichts-% hinzugefügt worden ist.

9. Mikroemulsion nach einem der vorherigen Ansprüche, der Holzschutzmittel, wie beispielsweise antimykotische Substanz(en) in einer Menge von 0,1 bis 5 Gewichts-% hinzugefügt worden ist.

10. Mikroemulsion nach einem der vorherigen Ansprüche, umfassend 12-26 Gewichts-% Ethanol.

11. Gel, umfassend 35-50 Gewichts-% der Mikroemulsion nach einem der Ansprüche 1-10 und 50 - 65 Gewichts-% eines mit Wasserstoff behandelten Pflanzenöls, bis zu einem Maximum oder einer Summe von Bestandteilen von 100 Gewichts-%.

12. Verfahren zur Herstellung einer Mikroemulsion nach einem der Ansprüche 1 bis 10, umfassend die folgenden aufeinanderfolgenden Schritte:
(a) Mischen von NaOH oder KOH mit Wasser, Glycerin und Ethanol, um eine Lösung zu erlangen;
(b) Mischen von Ölsäure, Tallölfettsäure und/oder gesättigten oder ungesättigten C₁₆-C₁₈-Fettsäuren in die Lösung, um eine Mikroemulsion zu erlangen;
(c) Mischen der Mikroemulsion mit flüssigem Kohlenwasserstoff(en) zu einer zweiten Mikroemulsion;
(d) optional Hinzufügen von Wasser oder Ethanol;
(e) optional Teilverdampfung des Ethanols,
wobei die Schritte (d) und (e) in umgekehrter Reihenfolge durchgeführt werden können.

13. Verfahren nach Anspruch 12, wobei in Schritt (d) Wasser oder Ethanol hinzugefügt wird.

14. Verfahren nach Anspruch 12, wobei in Schritt (e) Ethanol teilweise verdampft wird.

15. Verwendung einer Mikroemulsion nach einem der Ansprüche 1-10 zum temporären Korrosionsschutz auf Metalloberflächen, insbesondere Stahl.

16. Verwendung einer Mikroemulsion nach einem der Ansprüche 1-10 als Schmiermittel.

17. Verwendung nach Anspruch 16, wobei Antriebsketten, Kettensägeketten, Riemen, Drahtwinden, Kräne, Bremssättel, Kettensägeketten oder Blattfedern geschmiert werden.

18. Verwendung einer Mikroemulsion nach den Ansprüchen 1-10 als Metallbearbeitungsfluid.

19. Verwendung einer Mikroemulsion nach einem der Ansprüche 1-10 als Hydrophobierungsmittel für poröse Materialien.

20. Verwendung nach Anspruch 19, wobei die porösen Materialien Gesteinshohlräume und/oder Betonkonstruktionen darstellen.

21. Verwendung einer Mikroemulsion nach einem der Ansprüche 1-10 zum Beschichten von Papier, Pappe, Karton oder Gummi.

22. Verwendung einer Mikroemulsion nach einem der Ansprüche 1-10 als Leder- oder Gummiglanzflüssigkeit oder als Lederimprägnierflüssigkeit.

23. Verwendung einer Mikroemulsion nach einem der Ansprüche 1-10 als Verdickungsmittel für mit Wasserstoff behandeltes Pflanzenöl.

24. Verwendung eines Gels nach Anspruch 11 als Kraftstoff oder Schmiermittel.

25. Holzblock, **dadurch gekennzeichnet, dass** er mit einer Mikroemulsion nach einem der Ansprüche 1-10 imprägniert ist.

## Revendications

1. Microémulsion comprenant
de l'eau en une quantité de 1-30 % en poids ;
de l'oléate de sodium ou de potassium, des sels Na/K d'acide gras de tallol et/ou des sels Na/K d'acides gras saturés ou insaturés en C₁₆-C₁₈ en une quantité de 10-40 % en poids ;
de l'acide oléique, de l'acide gras de tallol ou des acides gras saturés ou insaturés en C₁₆-C₁₈ en une quantité de 2-40 % en poids ;
de l'éthanol en une quantité de 5-40 % en poids ;
du glycérol en une quantité de 5-40 % en poids ;
et un ou des hydrocarbure(s) liquide(s) en une quantité de 5-40 % en poids, jusqu'à un maximum ou un total de composants de 100 % en poids.

2. Microémulsion selon la revendication 1, comprenant de l'eau en une quantité de 2-10 % en poids ;
de l'oléate de sodium ou de potassium, des sels Na/K d'acide gras de tallol et/ou des sels Na/K d'acides gras saturés ou insaturés en C₁₆-C₁₈ en une quantité de 14-27 % en poids ;
de l'acide oléique, de l'acide gras de tallol ou des acides gras saturés ou insaturés en C₁₆-C ₁₈ en une quantité de 10-24 % en poids ;
de l'éthanol en une quantité de 5-40 % en poids, par exemple 5-26 % en poids ;
du glycérol en une quantité de 10-31 % en poids ;
et des hydrocarbures liquides en une quantité de 11-24 % en poids, jusqu'à un maximum ou un total de composants de 100 % en poids.

3. Microémulsion selon l'une quelconque des revendications précédentes, dans laquelle l'hydrocarbure liquide correspond à des alcanes et/ou des isoalcanes en C₁₁-C₂₀.

4. Microémulsion selon l'une quelconque des revendications précédentes, dans laquelle l'hydrocarbure liquide est l'hexadécane, le diesel, le HVO, le PAO, une huile de base naphténique ou paraffinique ou une combinaison de ceux-ci.

5. Microémulsion selon l'une quelconque des revendications précédentes, présentant une viscosité dans l'intervalle de 20 à 400 cp à 20 °C.

6. Microémulsion selon l'une quelconque des revendications précédentes, présentant une stabilité au stockage de plus de 30 jours à 20 °C.

7. Microémulsion selon l'une quelconque des revendications précédentes, à laquelle du goudron de bois a été ajouté en une quantité allant jusqu'à 15 % en poids.

8. Microémulsion selon l'une quelconque des revendications précédentes, à laquelle un antioxydant, un agent anti-usure et/ou un additif extrême-pression a été ajouté en une quantité de 0,1 à 5 % en poids.

9. Microémulsion selon l'une quelconque des revendications précédentes, à laquelle des produits de conservation du bois tels qu'une ou des substance(s) antifongique(s) ont été ajoutés en une quantité de 0,1 à 5 % en poids.

10. Microémulsion selon l'une quelconque des revendications précédentes, comprenant 12-26 % en poids d'éthanol.

11. Gel, comprenant 35-50 % en poids de la microémulsion selon l'une quelconque des revendications 1-10, et 50-65 % en poids d'une huile végétale hydrotraitée, jusqu'à un maximum ou total de composants de 100 % en poids.

12. Procédé de préparation d'une microémulsion selon l'une quelconque des revendications 1-10, comprenant les étapes consécutives suivantes :
(a) le mélange de NaOH ou de KOH avec de l'eau, du glycérol et de l'éthanol pour obtenir une solution ;
(b) le mélange d'acide oléique, d'acide gras de tallol et/ou d'acide(s) gras saturé(s) ou insaturé(s) en C₁₆-C₁₈ à la solution pour obtenir une microémulsion ;
(c) le mélange de la microémulsion avec un ou des hydrocarbures liquides pour former une seconde microémulsion ;
(d) éventuellement, l'addition d'eau ou d'éthanol ;
(e) éventuellement, l'évaporation partielle de l'éthanol, selon lequel les étapes (d) et (e) peuvent être effectuées dans l'ordre inverse.

13. Procédé selon la revendication 12, dans lequel, à l'étape (d), de l'eau ou de l'éthanol est ajouté.

14. Procédé selon la revendication 12, dans lequel à l'étape (e) l'éthanol est partiellement évaporé.

15. Utilisation d'une microémulsion selon l'une quelconque des revendications 1-10 pour la protection temporaire contre la corrosion sur des surfaces métalliques, en particulier l'acier.

16. Utilisation d'une microémulsion selon l'une quelconque des revendications 1-10 en tant que lubrifiant.

17. Utilisation selon la revendication 16, dans laquelle les chaînes d'entraînement, les chaînes de scie à chaîne, les courroies, les treuils à câble, les grues, les étriers de frein, les chaînes de scie à chaîne ou les ressorts à lames sont lubrifiés.

18. Utilisation d'une microémulsion selon les revendications 1-10 comme fluide de travail des métaux.

19. Utilisation d'une microémulsion selon l'une quelconque des revendications 1-10 comme agent d'étanchéité pour des matériaux poreux.

20. Utilisation selon la revendication 19, dans laquelle les matériaux poreux constituent des cavités rocheuses et/ou des constructions en béton.

21. Utilisation d'une microémulsion selon l'une quelconque des revendications 1-10 pour le revêtement de papier, de carton-pâte, de carton ou de caoutchouc.

22. Utilisation d'une microémulsion selon l'une quelconque des revendications 1-10 comme liquide de cirage pour cuir ou caoutchouc, ou comme liquide d'imprégnation du cuir.

23. Utilisation d'une microémulsion selon l'une quelconque des revendications 1-10, comme agent épaississant pour huile végétale hydrotraitée.

24. Utilisation d'un gel selon la revendication 11 comme carburant ou lubrifiant.

25. Bloc de bois, **caractérisé en ce qu'**il est imprégné d'une microémulsion selon l'une quelconque des revendications 1-10.
